# EUROPEAN PATENT APPLICATION

(11) **EP 1 391 503 A1**
(43) Date of publication of application: **25.02.2004**
(21) Application number: 02018014.7
(22) Date of filing: 12.08.2002
(51) Int. Cl.: C12N 5/06, C12N 5/08, A61K 35/56

(54) **A method of cell re-programming by cytoplasmic transfer**

(71) Applicant: Deutsches Krebsforschungszentrum Stiftung des öffentlichen Rechts, 69120 Heidelberg (DE)
(72) Inventor: Niehrs, Christof, Prof., 69117 Heidelberg (DE); Hansis, Christoph, Dr., 53115 Bonn (DE)
(74) Representative: Isenbruck, Günter, Dr.

(57) **Abstract**

The present invention refers to a method for reprogramming a recipient cell, the method comprising, first, contacting the recipient cell with the cytoplasm of a donor cell from an amphibian or from a bony fish and, second, detecting the expression of embryonic markers in the recipient cell. Furthermore, the present invention contemplates the therapeutic use of the disclosed method for diseases requiring the replacement or renewal of cells.

SEQUENCE LISTING

<110> Deutsches Krebsforschungszentrum, Stiftung des öffentlichen Rechts

<120> A method of cell re-programming by cytoplasmic transfer

<130> DK62035EP

<140> EP 02018014.7
<141> 2002-08-12

<160> 9

<170> PatentIn version 3.1

<210> 1
<211> 25
<212> DNA
<213> artificial sequence

<220>
<223> PCR Primer (Examples)

<220>
<221> misc_feature
<222> (1)..(25)
<223>

<400> 1
gacaacaatg aaaatcttca ggaga 25

<210> 2
<211> 23
<212> DNA
<213> artificial sequence

<220>
<223> PCR Primer (Examples)

<220>
<221> misc_feature
<222> (1)..(23)

<223>

<400> 2
ttctggcgcc ggttacagaa cca 23

<210> 3
<211> 14
<212> DNA
<213> artificial sequence

<220>
<223> PCR Primer (Examples)

<220>
<221> misc_feature
<222> (1)..(14)
<223>

<400> 3
gtctttccac cagg 14

<210> 4
<211> 21
<212> DNA
<213> artificial sequence

<220>
<223> PCR Primer (Examples)

<220>
<221> misc_feature
<222> (1)..(21)
<223>

<400> 4
ccatatcctg aggtggatca g 21

<210> 5

<211>  21
<212>  DNA
<213>  artificial sequence

<220>
<223>  PCR Primer (Examples)

<220>
<221>  misc_feature
<222>  (1)..(21)
<223>

<400>  5
cagggaggga aggtaatgag t                                                21

<210>  6
<211>  23
<212>  DNA
<213>  artificial sequence

<220>
<223>  PCR Primer (Examples)

<220>
<221>  misc_feature
<222>  (1)..(23)
<223>

<400>  6
cttggggaga gggggagtag cac                                              23

<210>  7
<211>  24
<212>  DNA
<213>  artificial sequence

<220>
<223>  PCR Primer (Examples)

<220>

<221>  misc_feature
<222>  (1)..(24)
<223>

<400>  7
gggggtcggg ggagcaggta ttgt                                              24

<210>  8
<211>  21
<212>  DNA
<213>  artificial sequence

<220>
<223>  PCR Primer (Examples)

<220>
<221>  misc_feature
<222>  (1)..(21)
<223>

<400>  8
cgtggggcgc cccaggcacc a                                                 21

<210>  9
<211>  22
<212>  DNA
<213>  artificial sequence

<220>
<223>  PCR Primer (Examples)

<220>
<221>  misc_feature
<222>  (1)..(22)
<223>

<400>  9
ttggccttgg ggttcagggg gg                                                22

## Description

The present invention refers to a method for reprogramming differentiated somatic cells into less differentiated cells by transfer of amphibian or fish cytoplasm and the therapeutic use of said method.

Stem cells are best described in the context of normal human development which begins when a sperm fertilizes an egg and creates a single cell that has the potential to form an entire organism. The fertilized egg is "totipotent", i.e. its potential is total. Approximately four days after fertilization and after several cycles of cell division, these totipotent cells begin to specialize, forming an hollow sphere of cells, called a blastocyste. Inside the hollow sphere, there is a cluster of cells called the inner cell mass. These inner cell mass cells are "pluripotent" - they can give rise to many but not all types of cells necessary for fetal development. The pluripotent stem cells undergo further specialization into stem cells that are committed to give rise to cells with a particular function. These more specialized stem cells are described as "multipotent". The latter stem cells are still present in the adult human body, but their potential to develop is less than those of pluripotent embryonic stem cells. Examples of adult stem cells include certain bone marrow cells that give rise to all blood lineages but can turn also into liver cells or cardiac muscle cells. Neural stem cells give rise to neurons and glial cells but can turn also into heart, lung or liver cells.

A potential application of human pluripotent cells is the generation of cells and tissue that could be used for so-called "cell therapies". Many diseases and disorders result from disruption of cellular function or destruction of tissues of the body. Varied organisms like e.g. salamanders, starfish, polyps and zebrafish can regenerate new heads, limbs, internal organs or other body parts if the originals are lost or damaged. Regenerating organisms take one of two approaches to replace a lost body part. Some, such as flatworms and the polyp *Hydra,* retain populations of stem cells throughout their lives, which are mobilized when needed. Unlike adult stem cells in human tissues, which are thought to have a relatively restricted capacity for developing into different cell types, these cells retain the ability to re-grow many of the body's tissues. Other organisms including newts, segmented worms and zebrafish, convert differentiated adult cells - which have stopped dividing and form part of the skin, muscle or another tissue - back into stem cells. This process is known as dedifferentiation.

In humans, ailing or destroyed tissue is often replaced by donated organs and tissues. Unfortunately, the number of people suffering from these disorders far outstrips the number of organs available for transplantation. Pluripotent stem cells, stimulated to develop into specialized cells, offer the possibility of a renewable source of replacement cells and tissue to treat myriad of diseases, conditions, and disabilities including Parkinson's and Alzheimer's diseases, spinal cord injury, stroke, skin alterations and diseases like e.g. burns or wrinkles, heart disease, diabetes, osteoarthritis and rheumatoid arthritis. However, if the full potential of donor embryo-derived cells lines is to be realized, the problem of donor-patient matching will need to be addressed. One approach, which avoids the problem of transplant rejection would be to generate stem cell lines from the patient's own cells through therapeutic cloning. The use of such so-called reprogrammed adult cells for cell therapies would reduce or even avoid the practice of using stem cells derived from human embryos of fetal tissue, sources that trouble many people on ethical grounds.

Several studies have shown that it is possible to re-program or dedifferentiate even mammalian somatic cells by taking the nucleus from an adult cell, transfer the nucleus to an oocyte devoid of maternal chromosomes, and achieve embryonic development directed by the transferred nucleus. The described technique named "nuclear transfer" was first performed with amphibians (Gurdon and Laskey, J. Embryol. Exp. Morphol. 1970 Sep;24(2):227-48) but has more recently been established in mammals as well. For example, Willadsen (Nature 1986 Mar 6-12;320(6057):63-5) described a procedure to investigate the development of sheep embryos in which whole blastomeres from 8- and 16-cell embryos were combined with enucleated or nucleated halves of unfertilized eggs. Robl et al performed nuclear transplantation in bovine embryos (J. Anim Sci. 1987 Feb;64(2):642-7) and re-programming was successfully done in nuclear transplant rabbit embryos (Stice and Robl, Biol Reprod. 1988 Oct;39(3):657-64).

These remarkable phenomena clearly indicate genetic programs that define cell differentiation are reversible and egg cytoplasm contains factors and activities to reprogram even terminally differentiated somatic nuclei so that they dedifferentiate and acquire pluripotency/totipotency required for normal development. However, the identities of these reprogramming factors are currently still to be defined.

The presently available methods for reprogramming of differentiated cells have distinct disadvantages. The transfer of somatic nuclei to embryos is technically very demanding in any animal and in humans it raises massive ethical objections. Cell-cell fusion on the other hand leads to aneuploid cells containing aberrant chromosome numbers, which are inherently different in character from normal cells and may be cancerous. Furthermore, they are immunologically non-compatible with the host, because one full set of chromosomes in the cell hybrid is of embryonic stem cell origin, typically derived from a foreign donor. Therefore it would be beneficial if other methods could be developed for converting differentiated cells to embryonic cell types, especially given their potential for producing different differentiated cell types for therapeutic use.

Recently it was shown that somatic cell extracts from primary human T cells or from a transformed T-cell line can be used to reprogram gene expression in differentiated nuclei such as 293T fibroblasts (Hakelien et al. Nat Biotechnol 2002, 20:460-6). Although in these experiments the fibroblasts acquired a phenotype characteristic for T cells, not embryonic cells, these experiments raise the possibility that an analogous method could be feasible to achieve embryonic reprogramming. WO 01/00650 discloses a method for re-programming mammalian somatic cells by the introduction of cytoplasm from bovine oocytes. However, the disclosed methods bear the disadvantage that bovine cytoplasm required for successful re-programming is only available in minute amounts since the oocytes are relatively small and difficult to obtain in larger quantities, as would be necessary for therapeutic use. Furthermore, in the described art, the cytoplasm is introduced into the recipient cell by complicated and, thus, expensive techniques like e.g. microinjection.

It is an object of the present invention to provide a method for reprogramming cells which avoids the drawbacks of the methods known in the art. The process should be suitable for use on a large scale and allow for an easy and fast introduction of the cytoplasm.

Preferably, the method should comprise a simple contacting step of the cells to be reprogrammed with the cytoplasm possessing reprogramming activities.

Accordingly, in a first aspect of the present invention there is provided a method for reprogramming a recipient cell comprising the following steps: (i) contacting the recipient cell with the cytoplasm of a donor cell from an amphibian or from a bony fish and (ii) detecting the expression of embryonic markers in the recipient cell.

As used herein, "contacting" means bringing the recipient cell and the cytoplasm of the donor cell in contact, namely under the time and temperature conditions being required for the reprogramming process. In this respect, contacting is often referred to "incubating".

In the context of the present invention, the amphibian belongs to the orders of Anura, Urodela or Gymnophiona, namely, for example, to the families (a) Bufonidae, such as Atelopus varius, Bufo alvarius, Bufo americanus, Bufo bufo, Bufo calamita, Bufo canorus, Bufo cognatus, Bufo fowleri, Bufo houstonensis, Bufo marinus, Bufo periglenes, Bufo quericus, Bufo terrestris, Bufo veraguensis, Bufo viridis, Bufo woodhousei; (b) Centrolenidae, such as Centrolenella fleishmannii; (c) Dendrobatidae, such as Dendrobates auratus, Dendrobates azurus, Dendrobates histrionicus, Dendrobates lehmannii, Phyllobates bicolor; (d) Discoglossidae, such as Bombina bombina, Bombina variegata, Alytes obstetricanus; (e) Hylidae, such as Acris crepitans Agalychnis callidryas, Corythomantis greeningi, Cyclorana platycephala, Gastrotheca ceratophrys, Hemophractus johnsoni, Hyla andersonii, Hyla arborea, Hyla avivoca, Hyla chrysoscelis, Hyla cinerea, Hyla gratiosa, Hyla regilla, Hyla squirella, Hyla versicolor, Litoria caerulea, Litoria chloris, Osteopilus septentrionalis, Pseudacris crucifer, Pseudacris regilla, Pseudacris triseriata; (f) the Hyperolidae family, such as Hyperolius viridiflavus; (g) Leptodactylidae, such as Leptodactylus pentadactylus, Syrrhophus marnocki, Telmatobius sp.; (h) Microphylidae, such as Gastrophryne carolinensis, Gastrophryne olivacea; (i) the Myobatrachidae family, such as Mixophyes schevilli, Rheobatrachus silus; (j) Pelobatidae, such as Pelobates fuscus, Megophrys nasuta, Scaphiopus bombifrons, Scaphiopus couchii, Scaphiopus holbrooki, Scaphiopus intermontanus (k) Pipidae, such as Pipa pipa, Pipa sp., Xenopus laevis, Xenopus tropicalis; (l) Pseudidae, such as Pseudis paradoxus; (m) Ranidae, such as Mantella aurantiaca, Rana arvalis, Rana aurora, Rana dalmatina, Rana esculenta, Rana ridibunda, Rana blairi, Rana cascadae, Rana catesbeiana, Rana clamitans, Rana grylio, Rana heckscheri, Rana okaloosae, Rana palustris, Rana pipiens, Rana septentrionalis, Rana sphenocephala, Rana sylvatica, Rana temporaria, Rana virgatipes; (n) Rhacophoridae, such as Rhacophorus schlegelii, Rhacophorus nigropalmatus; (o) Rhinophrynidae, such as Rhinophrynus dorsalis; (p) Ambystomatidae, such as Ambystoma annulatum, Ambystoma californiense, Ambystoma gracile, Ambystoma jeffersonianum, Ambystoma laterale, Ambystoma maculatum, Ambystoma opacum, Ambystoma talpoideum, Ambystoma texanum, Ambystoma tigrinum, Ambystoma tremblayi; (q) Amphiumidae, such as Amphiuma tridactylum; (r) Cryptobranchidae, such as Cryptobranchus alleganiensis; (s) Hynobiidiae, such as Hynobius nigrescens; (t) Plethodontidae, such as Aneides lugubris, Desmognathus fuscus fuscus, Desmognathus ochrophaeus, Ensatina eschscholtzii, Eurycea bislineata, Eurycea junaluska, Eurycea longicauda, Eurycea lucifuga, Eurycea nana, Eurycea sosorum, Eurycea wilderae; Gyrinophilus porphyriticus, Hemidactylium scutatum, Phaeognathus hubrichti, Plethodon cinereus, Plethodon cylindraceus, Plethodon glutinosus, Plethodon richmondi, Pseudotriton montanus, Pseudotriton ruber, Typhlomolge rathbuni; (u) Proteidae, such as Necturus maculosus; (v) Salamandridae, such as Cynops ensicauda, Chioglossa lusitanica, Cynops pyrrhogaster, Euproctes asper, Euproctes montanus, Notophthalmus viridescens, Salamandra salamandra, Taricha torosa, Triturus alpestris, Triturus vulgaris, Triturus cristatus, Triturus helveticus, Tylototriton shanjing; (w) Sirenidae, such as Pseudobranchus striatus, Siren intermedia, Siren lacertina; (x) Caecilidae, such as Afrocaecilia taitana, Gymnophis multiplicata; (y) Ichthyophiidae, such as Ichthyophis glutinosus; and (z) Typhlonectidae, such as Typhlonectes sp.

In a preferred embodiment of the present invention, the donor cell is isolated from an amphibian belonging to the Pipidae family. It is even more preferred if the donor cell providing the cytoplasm which is used for re-programming of somatic cells is isolated from Xenopus laevis.

Also included in the present invention are donor cells derived from the class of bony fishes (Osteichthyes), e.g. from the orders of Coelacanthidae, Ceratodiformes, Polypteriformes, Acipenseriformes Lepisosteiformes, Amiiformes, Elopiformes, Anguilliformes, Notacanthiformes, Clupeiformes, Osteoglossiformes, Mormyriformes, Salmoniformes, Stomiatiformes, Myctophiformes, Esociformes, Cetomimiformes, Ctenothrissiformes, Gonorynchiformes, Cypriniformes, Siluriformes, Percopsiformes, Batrachoidiformes, Gobiesociformes, Lophiiformes, Gadiformes, Atheriniformes, Beryciformes, Zeiformes, Lampridiformes, Gasterosteiformes, Channiformes, Synbranchiformes, Scorpaeniformes, Dactylopteriformes, Pegasiformes, Perciformes, Pleuronectiformes, Tetraodontiformes.

It is further preferred for the method of the present invention that the donor cell is an oocyte, e.g. an egg, or any other cell of an early developmental stage, including zygotes, embryonic stem cells, embryonic germ cells, primitive ectoderm-like cells or any other embryonic or adult stem cells being toti-, pluri- or multipotent. In a preferred embodiment of the present invention the donor cell is an oocyte.

In the context of the present invention, the recipient cell is terminally or partially differentiated and it can be of any suitable type and organism. The type and organism of the recipient cell can be different from type and organism of the donor cell. Preferably, the recipient cell is a somatic cell. Organisms possessing suitable recipient cells include but are not restricted to any vertebrate class like e.g. mammals, birds, amphibians, reptiles and fishes, with mammals being the preferred class. The mammal can for example be from the families of felidae, canidae, and/or other carnivora, muridae and/or other rodents, suidae, bovidae, equidae and/or other ungulates, pongidae and/or hominidae with recipient cells derived from humans being most preferred.

The recipient cell can be taken from any organ within any of said organisms, e.g. from heart, liver, lung, kidney, blood, brain, eye, pancreas, skin, stomach, intestine, bladder or bones. In addition, the term "recipient cell" refers to a variety of cell types, including epithelial and endothelial cells, fibroblasts, keratinocytes, mucosa cells and other skin cell types, leukocytes, erythrocytes, platelets, dendritic cells and other blood cells, astrocytes, oligodendrocytes, glial cells and other cells of the nerve system. In general, the method of the present invention can be applied to any recipient cell, whenever a source of cells in less differentiated state is desired, e.g. if the re-growth of a damaged tissue is favored.

The cytoplasm of the donor cell can be introduced into the recipient cell by any method known to a skilled artisan, e.g. by microinjection or by import via membrane-derived vesicles.

In a most preferred embodiment of the present invention the cytoplasm of the donor cell enters the recipient cell by itself without the provision of an external substance commonly used for the permeabilization of cells or without other manipulations like e.g, injection being necessary. The cytoplasm of the specific donor cells used in the present invention permits the penetration into the cell by itself. It is believed that this is due to the presence of certain compounds in the cytoplasm which permeabilize the plasma membrane of the recipient cell or which mediate the cellular uptake by endocytosis..

Furthermore, the cytoplasm of the donor cell can be introduced into the recipient cell by using a non-ionic detergent. This is also a preferred embodiment of the present invention.

The non-ionic detergent generally includes fatty alcohol ethoxylates, alkylphenol ethoxylates, fatty amine ethoxylate, fatty acid ethoxylate, fatty acid ester ethoxylate, alkanolamides, glycosidic tensides and aminoxides. The recipient cell is contacted with the cytoplasm of the donor cell in the presence of the non-ionic detergent under suitable buffer conditions allowing for a permeabilization of the recipient cell. Preferably the non-ionic detergent is a alkylphenol ethoxylate or a glycosidic tenside. More preferred is the use of a glycosidic tenside. Particular suitable for the transfer of cytoplasma is the use of the glycosidic tenside digitonin or a derivative thereof.

A suitable buffer, as used herein, can contain substances with buffering capacity such as HEPES, Tris, CAPSO, DIPSO, MOPS, MES, HEPPSO or PIPES, with HEPES being preferred, varied ionic salts such as potassium acetate or sodium acetate, chelating agents such as EGTA and diverse protease inhibitors.

The successful reprogramming of the recipient cell using the cytoplasmic extract of amphibian oocytes can be detected by methods known to the person skilled in the art, e.g. by determining the expression of suitable embryonic markers in the recipient cell. Suitable embryonic markers are known to the person skilled in the art; they include, but are not restricted to SSEA-3, SSEA-4, TRA-1-60, TRA-1-81, OCT-4, germ cell alkaline phosphatase and telomerase. Preferred embryonic markers of the present invention are OCT-4, germ cell alkaline phosphatase and telomerase.

The embryonic markers can be identified by methods well known to the person skilled in the art, including comparative PCR, RT-PCR, Southern Blotting, Northern blotting, subtractive hybridization, representational difference analysis (RDA), RNase protection assay, serial analysis of gene expression (SAGE) and mRNA differential display (DD) methods performing a scan for differentially expressed genes using cDNA microarray for hybridization studies. The preferred method to detect embryonic markers in the context of the present invention is RT-PCR.

In a further embodiment of the present invention oocyte cytoplasm is fractionated by methods known to the person skilled in the art. Egg cytoplasm contains factors and activities to reprogram even terminally differentiated somatic nuclei so that they dedifferentiate and acquire pluripotency/totipotency required for normal development. The fractions of the cytoplasm can further contain factors that mediate the permeabilization of the recipient cell. Feasible fractionation methods include e.g. salt (ammonium sulfate) fractionation, equilibrium density centrifugations exploiting e.g. percoll or sucrose gradients, chromatographic methods such as gel permeation chromatography using e.g. sephadex or sephacryl resins, capillary gas chromatography (CGC) or high performance liquid chromatography (HPLC), spectroscopic detection such as Fourier transform infrared, atomic emission detection, inductively coupled plasma detection, mass spectrometric techniques, such as e.g. matrix-assisted laser desorption/ionization time-of-flight mass spectrometry (MALDI-TOF MS).

One or more of the obtained fractions can be used for reprogramming of terminally or partially differentiated somatic cells, after having screened the fractions for their reprogramming capacity according to the requirements of the present invention.

Reprogramming and / or permeabilizing factors within the cytoplasm, or the fractions thereof, can be, among others, nucleic acids, in particular mRNA, proteins, proteoglycans, proteolipids and varied small molecules. It is possible to use the reprogramming and / or permeabilizing factors as such, after isolation and as mixtures thereof.

Nucleic acids bearing reprogramming or permeabilizing activity can be detected by comparative PCR, RT-PCR, Southern Blotting, Northern blotting, subtractive hybridization, representational difference analysis (RDA), RNase protection assay, serial analysis of gene expression (SAGE) and mRNA differential display (DD) methods using cellular extracts or fractions that do not possess reprogramming or permeabilizing activities for comparison. Methods to determine the amount and presence of proteins bearing reprogramming or permeabilizing activity comprise, among others, 2D-gel electrophoresis, Western blotting, immunoprecipitation, ELISA, and RIA using cellular extracts or fractions that do not possess reprogramming or permeabilizing activities for comparison.

The invention further contemplates a screening method for the purpose of identifying reprogramming and/or permeabilizing factors comprising the following steps: (i) contacting the recipient cell with the cytoplasm or a fraction thereof of a donor cell from an amphibian or from a bony fish and (ii) detecting the expression of embryonic markers in the recipient cell.

A further object of the present invention are pharmaceutical preparations which comprise an effective dose of the cytoplasm, a fraction thereof and/or at least one identified reprogramming factor, fragment or derivative thereof, and a pharmaceutically acceptable carrier, i.e. one or more pharmaceutically acceptable carrier substances and/or additives.

The cytoplasm, a fraction thereof or a reprogramming factor thereof, respectively the medicaments containing them, can be used for the treatment of diseases which require the replacement or renewal of certain cell types. Examples of such diseases comprise Parkinson's and Alzheimer's diseases, spinal cord injury, stroke, skin alterations and diseases like e.g. burns or wrinkles, heart disease, diabetes, osteoarthritis and rheumatoid arthritis.

In another aspect of the present invention, the nucleic acids which are identified of having reprogramming activity can be isolated by methods known to a skilled artisan and used to recombinantly express proteins encoded by said nucleic acids. The nucleic acids can thus be used for transfecting differentiated cells which are destined to be reprogrammed into a less differentiated for reasons obvious to the person skilled in the art.

Therefore, the present invention is also directed to a recombinant DNA and an expression vector which includes a nucleic acid displaying reprogramming activity operably linked to regulatory control nucleic acid which effects expression of the nucleic acid in a host cell. The present invention is further directed to a host cell which contains such a recombinant DNA or such an expression vector.

For the purpose of overexpression in differentiated cells, the nucleic acid can be placed in expression vectors capable of expressing an encoded protein, polypeptide or peptide. Nucleic acids encoding a desired protein or polypeptide are derived from cDNA or genomic clones using conventional restriction digestion or by synthetic methods, and are ligated into vectors from which they may be expressed. Vectors may, if desired, contain nucleic acids encoding portions of other proteins, thereby providing a fusion protein.

Expression vectors include plasmids designed for the expression of proteins or polypeptides fused to or within bacterial phage coat proteins. The DNA encoding the desired protein or polypeptide, whether in a fusion, premature or mature form, may be ligated into expression vectors suitable for any host. The DNA encoding the desired polypeptide may also contain a signal sequence to permit secretion from the intended host. Both prokaryotic and eukaryotic host systems are contemplated. Preferably the host cells are of mammalian, most preferably they are of human origin.

The host cells being transformed by the methods described as well as the recombinant DNA and / or the vector can be components of a kit which is used for treatment of disorders requiring the regrowth or the renewal of cells. Examples of such disorders include diseases requiring replacement or renewal of cells, preferably Parkinson's disease, Alzheimer's disease, spinal cord injury, stroke, skin alterations and diseases like e.g. burns or wrinkles, heart disease, diabetes, osteoarthritis and rheumatoid arthritis.

The present invention includes furthermore embryonic stem cells possessing toti- or pluropotency which have been obtained by reprogramming differentiated cells, preferably human cells, by applying the methods of the present invention. Such cells can be used for therapeutic purposes, i.e. they can be used as a source to give rise to any differentiated cell type, which can be used for cell transplantation therapies.

### DESCRIPTION OF THE FIGURES:

### Figure 2a

### Expression of Oct-4, Germ Cell Alkaline Phosphatase (GCAP), Natriuretic Peptide Receptor Type A (NPR-A), β-actin mRNA in human 293T cell spheres

Panel 1 shows amplification for *Oct-4* (38 cycles), panel 2 for *Germ Cell Alkaline Phosphatase (GCAP,* 44 cycles), panel 3 for *Natriuretic Peptide Receptor Type A (NPR-A,* 41 cycles). Samples were normalized for their *β-actin* expression as shown in panel 4 (24 cycles). Lane 1 = 293T spheres treated with Digitonin (+) and egg extract (+); lane 2 = Digitonin +, egg extract -; lane 3 = Digitonin -, egg extract +; lane 4 = Digitonin -, egg extract -; lane 5 = untreated control cells. Lanes 6-10 correspond to lanes 1-5, but without Superscript II Reverse Transcriptase in the cDNA synthesis. Lane 11 = egg extract as template, lane 12 = water control. 10µl samples each were loaded onto 1.8% agarose gels with 1xTBE.

*Oct-4* and *GCAP* are overexpressed in egg extract treated spheres compared to spheres not treated with egg extract and control cells. Complementary to this pattern, *NPR-A* is overexpressed in spheres not treated with egg extract and in control cells compared to egg extract treated spheres.

### Figure 2b

### Expression of Oct-4, β-actin mRNA in mouse NIH 3t3 cell spheres

The right panel shows amplification for *Oct-4 (47* cycles). Samples were normalized for their *β-actin* expression as shown in the left panel (30 cycles). For both panels: lane 1 = NIH 3T3 spheres treated without Digitonin (-) and with egg extract (+); lane 2 = Digitonin -, egg extract -; lane 3 = untreated control cells. 10µl samples each were loaded onto 1.8% agarose gels with 1xTBE.

*Oct-4* is overexpressed in the egg extract treated sphere compared to the sphere not treated with egg extract and control cells.

### Figure 2c

### Expression of Oct-4, Germ Cell Alkaline Phosphatase (GCAP) and β-actin mRNA in human leukocyte spheres

Panel 1 shows amplification for *Oct-4* (38 cycles) and panel 2 for *Germ Cell Alkaline Phosphatase (GCAP, 44* cycles). Samples were normalized for their *β-actin* expression as shown in panel 3 (24 cycles). Lane 1 = leucocyte spheres treated with Digitonin (+) and egg extract (+); lane 2 = Digitonin +, egg extract -; lane 3 = Digitonin -, egg extract +; lane 4 = Digitonin -, egg extract -; lane 5 = untreated control cells. Lanes 6-10 correspond to lanes 1-5, but without Superscript II Reverse Transcriptase in the cDNA synthesis. Lane 11 = egg extract as template, lane 12 = water control. 10µl samples each were loaded onto 1.8% agarose gels with 1xTBE.

*Oct-4* and *GCAP* are overexpressed in egg extract treated spheres compared to spheres not treated with egg extract and control cells.

### EXAMPLES:

### A. Production of Xenopus laevis egg extract

The production of *Xenopus laevis* egg extract essentially followed a protocol by Leno, 1998. Frogs were injected with 600IU hCG at 21:00h the day before the preparation. The following morning eggs were squeezed in glass petri dishes, one petri dish per frog. The eggs were dejellied with 2% cysteine HCI, freshly prepared with NaOH to pH 7.8, and 10min rocking at room temperature. Afterwards eggs were washed three times with 1x Barth solution in plastic beakers, misshape eggs removed and eggs transferred to 50ml Falcon tubes on ice. Eggs were then washed two times with ice cold Extraction Buffer (50mM HEPES/KOH, pH 7.4, 50mM KCl, 5mM MgCl₂, 2mM 2-mercaptoethanol, 5mM EGTA, second wash with 10µg/ml each Cytochalasin B, Leupeptin, Aprotinin, Pepstatin A). Eggs were spun at 1000rpm for 1min, excessive buffer was carefully removed and the eggs transferred to 2ml Eppendorf tubes. Eggs were crushed by centrifugation at 10.000g for 15min at 4°C, the middle layer sucked out with a pipette tip and transferred to a new tube. This centrifugation step was repeated with 16.000g to clean the extract. Afterwards 2% glycerol was added, 20µl samples snap-frozen in liquid nitrogen and stored at -80°C.

### B. Treatment of 293T, NIH 3T3 cells and human leukocytes with digitonin and Xenopus egg extract

293T or NIH 3T3 cells were grown to sub-confluency in DMEM + 10% FCS +1% Glutamine and 10% CO₂, cells washed out of the culture flask by PBS and transferred to 15ml Falcon tubes. Cells were then centrifuged at 500rpm for 5min (same conditions for all further centrifugation steps) and resuspended in 1ml ice cold Transport Buffer (Adam et al., 1990; 20mM HEPES, pH 7.3, 110mM KAc, 5mM NaAc, 2mM MgAc, 1mM EGTA, 2mM DTT, 1 µg/ml each Aprotinin, Leupeptin, Pepstatin A). 500ml human blood samples, obtained from HIV, HBV, HCV negative donors, were ca. 5 times enriched for leukocytes by removing plasma and erythrocytes after centrifugation in donor bags. The leukocyte-enriched blood sample was diluted 1:1 with PBS and 25ml laid over 16ml Ficoll in a 50ml Falcon tube. After centrifugation for 10min at 2000rpm with deactivated breaks, ca. 1ml of the purified buffy coat layer with leukocytes was removed by pipetting and transferred into a 15ml Falcon tube. The tube was filled up with PBS, centrifuged for 10min at 2000rpm, the supernatant discarded and 0.5ml ice cold Transport Buffer added to the pellet. Cells were centrifuged again, resuspended in 1ml Transport Buffer + 35µg/ml digitonin (from 20mg/ml stock solution in DMSO) and incubated on ice for 5min. Cells were centrifuged, resuspended in 1ml Transport Buffer, centrifuged and resuspended in 40µl Incubation Mix (2µl 20mg/ml BSA, 2µl 20mM ATP, 2µl 100mM Phosphocreatine, 2µl 400U/ml Creatine Kinase, 2µl RNAsin, 10µl Transport Buffer, 20µl egg extract) for 30min at 37°C. Incubation was terminated by addition of 1ml ice cold Transport Buffer, cells centrifuged, resuspended in DMEM + 10% FCS +1% Glutamine, plated out in 4 cm petri dishes and kept at 10% CO₂. The appearance of spheres was assessed at day 5 (day 1 = experiment) and documented by photography. Spheres were transferred to agarose coated petri dishes later to prevent attachment at the bottom. Control experiments were performed without digitonin (incubation in Transport Buffer only), without egg extract (incubation in Incubation Mix with Transport Buffer replacing egg extract) or without digitonin and egg extract.

### C. RT-PCR for embryonic markers Oct-4, Germ Cell Alkaline Phosphatase, Telomerase

### RNA preparation

RNA preparation of spheres and clusters with or without digitonin treatment, with or without egg extract or untreated control cells essentially followed a protocol developed by Rappolee at al., 1989. Spheres, cell clusters and untreated control cells were harvested at day 5-18 by glass needle aspiration and transferred to 100µl Denaturing Solution (4M GnSCN, 0.5% Sarcosinate, 1% β-mercaptoethanol, 20µg rRNA), laid over 100µl 5.7M CsCl and spun for 3.5h at 70.000rpm in an Optima TL Tabletop Ultracentrifuge (Beckman Coulter, Fullerton, CA, USA) in a TL 100.2 rotor. The supernatant was discarded in several steps with new pipette tips each time to avoid DNA contamination. The pellet was then washed in 70% EtOH, dried, resuspended in 20µl H₂O DEPC, transferred to an Eppendorf tube and 6µl 2M NaAc pH 4.0 and 60µl 100% ice cold EtOH added. The samples were precipitated at 4°C overnight and the pellet washed with 70% EtOH.

### DNAse digestion

The dried pellet was resuspended in 1.3µl H₂O DEPC and 0.18µl 10x Reaction Buffer (all reagents Invitrogen Life Technologies, Carlsbad, CA, USA) and 0.3µl *DNAse I* were added and incubated for 15min at room temperature. The *DNAse* digestion was stopped by addition of 0.18µl EDTA and heat inactivation at 65°C for 15min.

### RT reaction

Four microliter 50ng/µl random hexamers (all reagents Invitrogen) were added to the *DNAse* treated RNA, incubated at 70°C for 10min and immediately placed on ice. Then 1µl 10x PCR buffer, 1µl 25mM MgCl₂, 0.5µl 10mM dNTP, 1µl 0.1M DTT, 0.1µl RNAsin (40U/µl) were added and incubated at 25°C for 5min. 2µl of the sample were transferred to a new tube for RT negative control reactions; to the rest 0.4µl *Superscript II* RT was added, incubated at 25°C for 10min and at 42°C for 50min. The reaction was terminated by heating to 95°C for 5min.

### PCR

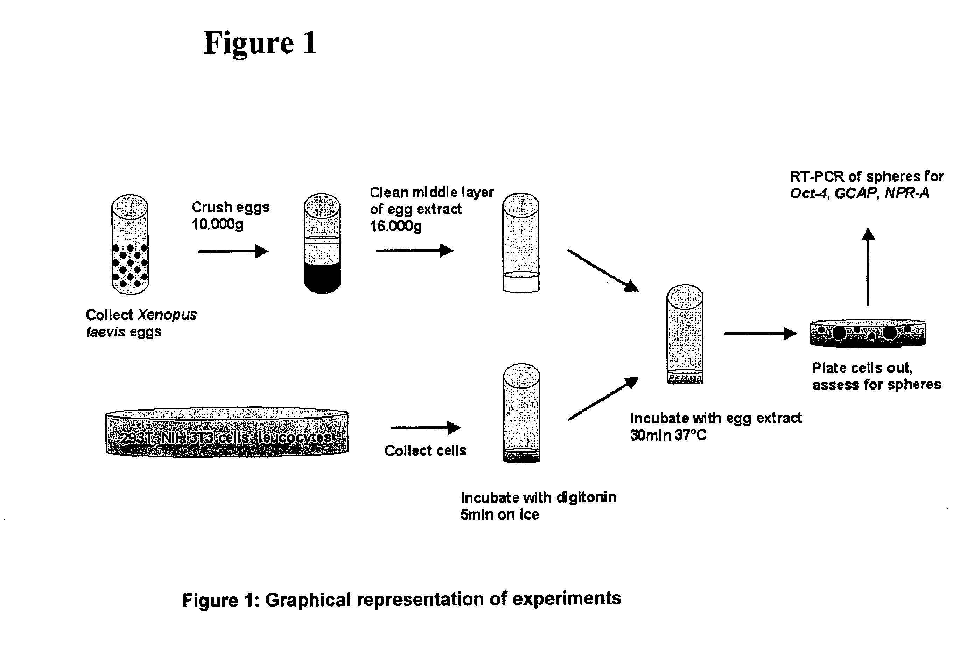

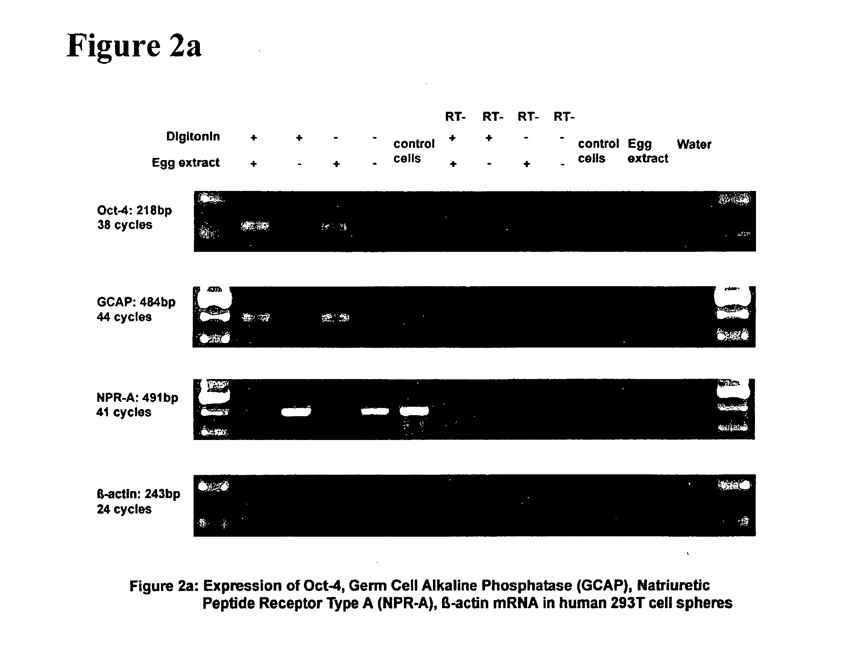

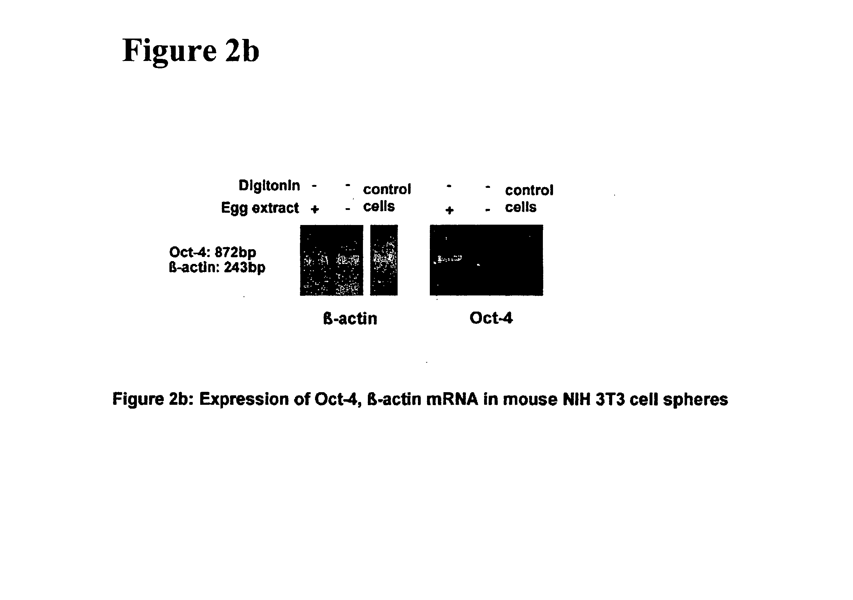

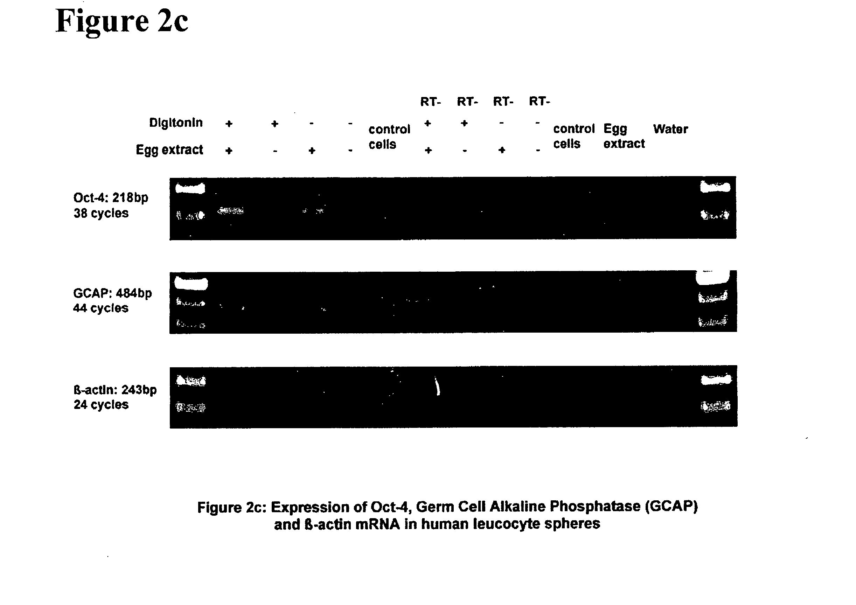

PCR reactions were performed using 0.6µl undiluted-0.3µl 1:100 diluted cDNA template as determined by normalization for β-actin expression at 24 or 30 cycles (see Figure 2a, b, c). To the cDNA in a 0.2ml Eppendorf tube 500nM primers (0.5µl each) for *Oct-4* (modified after Abdel-Rahman et al., 1995; for human cells: Inner 5': GACAACAATGAAAATCTTCAGGAGA, Inner 3': TTCTGGCGCCGGTTACAGAACCA; for mouse cells: mOct4a o5' GTCTTTCCACCAGG and Inner 3'), *Germ Cell Alkaline Phosphatase* (Hung et al., 2001; GCAP 1: CCATATCCTGAGGTGGATCAG, GCAP 2: CAGGGAGGGAAG GTAATGAGT), *Natriuretic Peptide Receptor Type A* (Ortego and Coca-Prados, 1999; NPR-A 5': CTTGGGGAGAGGGGGAGTA GCAC; NPR-A 3': GGGGGTCGGGGGAGCAGGTATTGT) and β-actin (5': CGTGGGGCGCCCCAGGCACCA; 3': TTGGCCTTGGGGTTCAGGGGGG) and 47µl of a mastermix containing 1x PCR buffer (all reagents Roche Diagnostics, Mannheim, Germany, except dNTP: MBI Fermentas, St.Leon-Rot, Germany) 2mM MgCl₂, 20µM dNTP and 37.9µl H₂O were added. The samples were heated to 94°C, 1µl *Taq* Polymerase added and 30-47 cycles performed with 94°C for 15sec, 62°C for 30sec and 72°C for 30sec followed by 72°C for 10min and 4°C for 5min (see Figure 2a, b, c) in a PTC-200 thermal cycler (MJ Research, Watertown, MA, USA). Control reactions included RT negative samples, H₂O samples and egg extract.

## Claims

1. A method for reprogramming a recipient cell comprising the following steps: (i) contacting the recipient cell with the cytoplasm of a donor cell from an amphibian or from a bony fish and (ii) detecting the expression of embryonic markers in the recipient cell.

2. A method according to claim 1, wherein the donor cell is an oocyte or an embryonic cell.

3. A method according to claim 1 or 2, wherein the amphibian is selected from the group of (a) the Bufonidae family, (b) the Centrolenidae family, (c) the Dendrobatidae family, (d) the Discoglossidae family, (e) the Hylidae family, (f) the Hyperolidae family, (g) the Leptodactylidae family, (h) the Microphylidae family, (i) the Myobatrachidae family, (j) the Pelobatidae family, (k) the Pipidae family, preferably Pipa pipa, Pipa sp., Xenopus laevis, Xenopus tropicalis; (1) the Pseudidae family, (m) the Ranidae family, (n) the Rhacophoridae family, (p) the Ambystomatidae family, (q) the Amphiumidae family, (r) the Cryptobranchidae family, (s) the Hynobiidiae family, (t) the Plethodontidae family, (u) the Proteidae family, (v) the Salamandridae family, (w) the Sirenidae family, (x) the Caecilidae family, (y) the Ichthyophiidae family, and (z) the Typhlonectidae family.

4. A method according to any of claims 1 to 3, wherein the amphibian belongs to the family of Pipidae.

5. A method according to any of claims 1 to 4, wherein the amphibian is Xenopus laevis.

6. A method according to claim 1 or 2, wherein the bony fish is selected from the group consisting of Coelacanthidae, Ceratodiformes, Polypteriformes, Acipenseriformes Lepisosteiformes, Amiiformes, Elopiformes, Anguilliformes, Notacanthiformes, Clupeiformes, Osteoglossiformes, Mormyriformes, Salmoniformes, Stomiatiformes, Myctophiformes, Esociformes, Cetomimiformes, Ctenothrissiformes, Gonorynchiformes, Cypriniformes, Siluriformes, Percopsiformes, Batrachoidiformes, Gobiesociformes, Lophiiformes, Gadiformes, Atheriniformes, Beryciformes, Zeiformes, Lampridiformes, Gasterosteiformes, Channiformes, Synbranchiformes, Scorpaeniformes, Dactylopteriformes, Pegasiformes, Perciformes, Pleuronectiformes, Tetraodontiformes.

7. A method according to any of claims 1 to 6, wherein no external permeabilizing reagents are used to allow for the cytoplasm of the donor cell to enter the recipient cell.

8. A method according to any of claims 1 to 6, wherein a non-ionic detergent selected from the group of fatty alcohol ethoxylates, alkylphenol ethoxylates, fatty amine ethoxylate, fatty acid ethoxylate, fatty acid ester ethoxylate, alkanolamides, glycosidic tensides and aminoxides is used to allow for the cytoplasm of the donor cell to enter the recipient cell.

9. A method according to claim 8, wherein the non-ionic detergent is selected from the group alkylphenol ethoxylates and glycosidic tensides.

10. A method according to claims 8 and 9, wherein the glycosidic tenside is digitonin.

11. A method according to any of claims 1 to 10, wherein the recipient cell is a mammalian cell.

12. A method according to claim 11, wherein the mammalian cell is a human somatic cell.

13. A fraction of cytoplasm from amphibian or bony fish oocytes containing at least one reprogramming and / or permeabilizing factor.

14. A reprogramming and / or a permeabilizing factor contained in the cytoplasm from amphibian or bony fish oocytes or contained in the fraction according to claim 13.

15. A factor according to claim 14, wherein said factor is a nucleic acid or a protein.

16. A method for identifying reprogramming and/or permeabilizing factors comprising the following steps: (i) contacting the recipient cell with the cytoplasm of a amphibian or bony fish oocytes or a fraction according to claim 13 and (ii) detecting the expression of embryonic markers in the recipient cell.

17. The cytoplasm of amphibian or bony fish oocytes and / or a fraction according to claim 13 and / or at least one reprogramming and / or permeabilizing factor according to claim 14, a fragment or derivative thereof for use as a pharmaceutical.

18. The use of the cytoplasm from amphibian or bony fish oocytes and / or a fraction according to claim 13 and / or at least one reprogramming and / or permeabilizing factor according to claim 14 for the manufacture of a medicament for the treatment of a disease requiring replacement or renewal of cells, preferably Parkinson's disease, Alzheimer's disease, spinal cord injury, stroke, skin alterations and diseases like burns or wrinkles, heart disease, diabetes, osteoarthritis and rheumatoid arthritis.

19. A pharmaceutical preparation, preferably for the treatment of a disease requiring replacement or renewal of cells, comprising the cytoplasm from amphibian or bony fish oocytes and / or a fraction according to claim 13 and / or at least one reprogramming and / or permeabilizing factor according to claim 14 and a pharmaceutically acceptable carrier.

20. A recombinant DNA comprising a nucleic acid as specified in claim 15, operably linked to regulatory control nucleic acid sequences which can affect expression of said nucleic acid sequences in a host cell.

21. An expression vector or plasmid comprising the recombinant DNA according to claim 20.

22. A host cell comprising the expression vector of claim 21.

23. A host cell according to claim 22, wherein the host cell is a eukaryotic, preferably a human cell.

24. A kit comprising the recombinant DNA according to claim 20 and/or the vector according to claim 21 and/or the host cell according to claim 22 for use in the treatment of diseases requiring replacement or renewal of cells, preferably Parkinson's disease, Alzheimer's disease, spinal cord injury, stroke, skin alterations and diseases like burns or wrinkles, heart disease, diabetes, osteoarthritis and rheumatoid arthritis.

25. A human somatic cell being reprogrammed according to the method of any of claims 1 to 12.
